# EUROPEAN PATENT APPLICATION

(11) **EP 3 524 251 A1**
(43) Date of publication of application: **14.08.2019**
(21) Application number: 19167461.3
(22) Date of filing: 13.11.2013
(51) Int. Cl.: A61K 31/5377, A61P 25/00

(54) **METHOD OF TREATMENT OF AGGRESSION**

(30) Priority: 13.11.2012 US 201261725883 P; 16.11.2012 US 201261727570 P
(62) Divisional of application: 13798833.3
(71) Applicant: Supernus Pharmaceuticals, Inc., Rockville, MD 20850 (US)
(72) Inventor: HAMM, Adam Kenneth., Wake Forest NC 27587 (US)
(74) Representative: Louis Pöhlau Lohrentz

(57) **Abstract**

The current invention offers a method of treatment of aggression in a human subject suffering from ADHD, comprising: (a) determining the weight of the human subject; (b) calculating a dose of molindone such to achieve a plasma concentration, based on body weight, that does not saturate the molindone receptors; (c) administering the dose of step (b) to the mammalian subject.

## Description

### BACKGROUND

Aggression and similar syndromes, including impulsivity and irritability, represent a broad category of behaviors that complicate the management of several disease states, such as attention deficit hyperactivity disorder (ADHD), bipolar disorder, autism, and post traumatic stress disorder. In some cases, 25-50% of patients optimally treated for the underlying disorder continue to manifest these syndromes (J Am Acad Child Adolesc Psychiatry, 2007 Mar; 46(3):309-22).

All of the publications, patent applications and patents cited in this specification are incorporated herein by reference in their entirety.

Dopaminergic therapies are among the most prescribed for these behavioral syndromes, and include such molecules as haloperidol and other antipsychotics. The dopamine receptors for these molecules are grouped into two families: the D1, which includes the D1 and D5 receptors, and the D2, which includes the D2, D3 and D4 receptors. The two families differ by the manner in which the receptor protein is incorporated into the cell membrane, and by the pharmacology of the molecules that have an affinity for each type. Each receptor type is a distinct entity with its unique gene, anatomy in the brain, and affinity for different molecules. Some dopamine receptor subtypes, such as the D2 receptor, have further modifications in the protein structure, giving rise to further sub-classification, e.g., D2ₛₕₒᵣₜ and D2_{long}.

There is increasing evidence that D5 receptor activity would be beneficial in the treatment of aggression and similar behavioral syndromes.

The D5 receptor has very specific localization in the brain, and is found in such areas as the parafascicular nucleus of the thalamus, as well as the prefrontal cortex, hippocampus, ventral tegmental area, substantia nigra and raphe nucleus (Hartman DS, Civelli O. Molecular attributes of dopamine receptors: new potential for antipsychotic drug development. Ann Med 1996; 28(3):211-9). The parafascicular nucleus is involved in the behavioral process of attention to critical sensory input and activation of the subject toward that stimulus. One of the important paradigms in which the parafascicular nucleus participates is the activation of the fight or flight response. Therefore, the parafascicular nucleus is likely involved in activating early components of aggressive behavior (Matsumoto N, Minamimoto T, Graybiel AM, Kimura M. Neurons in the thalamic CM-Pf complex supply striatal neurons with information about behaviorally significant sensory events. J Neurophysiol 2001;85(2):960-76).

The gene for the D5 receptor, DRD5, is associated with impulsiveness and with symptomology associated with disruptive behavioral disorders, such as antisocial personality disorder (Vanyukov MM, Moss HB, Kaplan BB, Kirillova GP, Tarter RE. Antisociality, substance dependence, and the DRD5 gene: a preliminary study. Am J Med Genet 2000;96(5):654-8). DRD5 is also associated with genetic transmission of a number of disorders associated with aggression, irritability and impulsivity, including schizophrenia, Tourette's, and ADHD (Maher BS, Marazita ML, Ferrell RE, Vanyukov MM. Dopamine system genes and attention deficit hyperactivity disorder: a meta-analysis. Psychiatr Genet 2002;12(4):207-15). Blockade of the D5 receptor in a knockout model is associated with decreased motor activity, which may be akin to decreased aggression (Holmes A, Hollon TR, Gleason TC, et al. Behavioral characterization of dopamine D5 receptor null mutant mice. Behav Neurosci 2001;115(5): 1129-44).

Molindone is a typical antipsychotic drug that has high affinity for the D2 family of dopamine receptors, where it is thought to exert its therapeutic action. Molindone was previously suggested for the treatment of aggression in both adult and pediatric patients (Greenhill LL, Barmack JE, Spalten D, Anderson M, Halpern F. Molindone Hydrochloride in the treatment of aggressive, hospitalized children [proceedings]. Psychopharmacol Bull 1981;17(1):125-7; Itil TM, Wadud A. Treatment of human aggression with major tranquilizers, antidepressants, and newer psychotropic drugs. J Nerv Ment Dis 1975;160(2-1):83-99). Molindone was also evaluated for children with the early-onset schizophrenia spectrum disorders (J Am Acad Child Adolesc Psychiatry, 2007, August, 46:8, p.969 - 978 and Am J Psychiatry, 165:11, Nov. 2008). WO 2010/080603 describes the use of molindone for the treatment of aggression, the disclosure of which is incorporated herein in its entirety by reference.

For adults with schizophrenia, the dose of molindone may range from 100 to 225mg per day (Bagnall A, Fenton M, Kleijnen J, Lewis R. Molindone for schizophrenia and severe mental illness. Cochrane Database Syst Rev 2007(1):CD002083). In general, the dose of other antipsychotics used for the treatment of aggressive behavior are about 50% relative to those used for the treatment of psychosis in schizophrenia (J Am Acad Child Adolesc Psychiatry. 2006 Jul;45(7):792-800).

### SUMMARY OF THE INVENTION

The current invention offers a method of treatment of aggression in a human subject suffering from ADHD, Tourette's and/or autism, comprising: (a) determining the weight or age of the human subject; (b) calculating a dose of molindone needed to achieve a plasma concentration, or other parameter (e.g., brain concentration), based on body weight or age, that does not saturate the molindone receptors; (c) administering the dose of step (b) to the human subject. The molindone receptors may comprise D2 receptors, D5 receptors, or both, or others. The dose may be calculated such that the molindone administered would comprise less than 90%, 80%, 70%, 60%, 50%, even less than 20% of the molindone dose required for treatment of schizophrenia.

The invention also provides a method of treating aggression in a human subject suffering from ADHD, Tourette's and/or autism comprising administering a daily dose of molindone between 15 mg and 60 mg for human subjects weighing over 30 kg and a daily dose of molindone less than 25mg for human subjects weighing less than 30 kg.

### DETAILED DESCRIPTION OF THE PREFERRED EMBODIMENTS

Unless otherwise specified, "a" or "an" means "one or more."

A Phase IIb study was conducted for the treatment of impulsive aggression in Attention Deficit and Hyperactivity Disorder (ADHD) patients with a pharmaceutical preparation of molindone. The study was a multi-center randomized, double-blind, placebo controlled clinical trial in children 6 to 12 diagnosed with ADHD and characterized by impulsive aggression that is not controlled by optimal stimulant and psychosocial treatment. It was a dose finding study with the primary objective of identifying an effective dose range in children of different weight or age groups. The children were divided into two age groups (*i.e*., less than 30 kg and greater than or equal to 30 kg). The low, medium, and high doses for the under 30 kg group were 12 mg, 24 mg, and 36 mg, respectively. Similarly, the low, medium, and high doses for the 30 kg and over group were 18 mg, 36 mg, and 54 mg, respectively.

Three doses of molindone were studied with the primary endpoints of (a) effect in reducing impulsive aggression (measured with change in the score of the Retrospective - Modified Overt Aggression Scale "R-MOAS" at the end of the study from the baseline), and (b) rate of remission of aggression, after at least three weeks of treatment. Secondary objectives included safety and tolerability of molindone as well as the effect on the Clinical Global Impression and the Oppositional Defiant Disorder subscale score of the SNAP-IV Questionnaire and other endpoints [e.g., SNAP, clinical labs, AEs, etc.]. Patients who completed the study were offered the opportunity to continue into an open-label phase of six months duration.

The results varied by weight group of treated patients and by dose levels. For patients of weight 30 kg or more, the low (18 mg) and medium (36 mg) doses of molindone, but not the high (54 mg) dose showed statistical significance vs placebo on the change in R-MOAS primary endpoint with p-values of 0.024 and 0.049 for the low and medium doses, respectively (Example 1). In addition, both doses resulted in remission of aggression with statistical significance vs placebo with p-values of 0.004 and 0.021, respectively (Example 2). Finally, the low dose met all secondary endpoints of Clinical Global Impression for severity and improvement, and of Oppositional Defiant Disorder with statistical significance vs placebo with p-values of 0.007, 0.017 and 0.039, respectively. The high dose did not show efficacy across any of the measures.

It was unexpectedly discovered that for patients under 30 kg in weight the studied doses did not show statistical significance vs placebo on the R-MOAS endpoint. Coupled with the fact that the high dose did not show efficacy in the over 30 kg cohort, this observation indicates that the most effective doses are those that achieve certain plasma concentrations, exposure levels, or other parameters that do not exceed a level beyond which a saturation threshold is reached.

The low (12 mg or 18 mg) and medium (24 mg or 36 mg) doses of molindone met the efficacy endpoint of rate of remission of aggression for all patients with statistical significance vs placebo and p-values of 0.009 and 0.043, respectively. The low and medium doses showed a reduction in score for the R-MOAS with p-values of 0.071 and 0.115. The clear and consistent trend for both arms reinforces the statistically significant remission scores. Furthermore, the magnitude of the score reductions seen in both arms was in a range that would be clearly clinically significant in patients.

Molindone was well tolerated throughout the study across all doses. The patients may also suffer from Tourette's and/or autism. Preferable ranges for low, medium, and high doses for the under 30 kg group are 10 - 14 mg, 22-26 mg, and 34-38 mg, respectively. Similarly, preferable ranges for the low, medium, and high doses for the 30 kg and over group are 16 - 20 mg, 34-38 mg, and 52-56 mg, respectively.

### EXAMPLES

### Example 1

**Table 1. R-MOAS ratings - Mean Changes from Baseline, Low Weight Subgroup (< 30 kg)**

| Primary Efficacy Variable: R-MOAS (LOCF)¹ | | Treatment group | | | |
|---|---|---|---|---|---|
| | | Placebo | Low Dose | Medium Dose | High Dose |
| Number of Patients | | 12 | 12 | 15 | 14 |
| Baseline (visit 5), mean (SD) | | 56.9 (20.58) | 59.3 (30.22) | 69.3 (33.12) | 48.6 (28.80) |
| End of treatment (visit 10), mean (SD) | | 36.8 (36.38) | 34.2 (27.29) | 38.5 (36.70) | 32.2 (24.14) |
| Change from Baseline, mean (SD) | | -20.1 (27.11) | -25.1 (23.00) | -30.7 (31.22) | -16.4 (36.90) |
| Treatment vs Placebo | P-value | | 0.729 | 0.643 | 0.997 |
| | 95% CI² | | (-26.72, 18.83) | (-26.93, 16.79) | (-22.10, 22.01) |

| | | | | | |
|---|---|---|---|---|---|
| 1. LOCF = Last Observation Carried Forward 2. CI = Confidence Interval | | | | | |

**Table 2. R-MOAS ratings - Mean Changes from Baseline, High Weight Subgroup (≥ 30 kg)**

| Primary Efficacy Variable: R-MOAS (LOCF) | | Treatment group | | | |
|---|---|---|---|---|---|
| | | Placebo | Low Dose | Medium Dose | High Dose |
| Number of Patients | | 18 | 15 | 15 | 17 |
| Baseline (visit 5), mean (SD) | | 44.3(21.26) | 51.5 (31.29) | 54.4 (32.00) | 51.8 (26.73) |
| End of treatment(visit 10), mean(SD) | | 25.9 (24.48) | 9.8 (13.01) | 13.5(15.71) | 28.8 (40.60) |
| Change from Baseline, mean(SD) | | -18.3(17.67) | -41.7(32.48) | -40.9(34.84) | -23.0 (27.55) |
| Treatment vs Placebo | P-value | | 0.024 | 0.049 | 0.966 |
| | 95% CI | | (-35.91, -2.58) | (-33.54, -0.07) | (-16.48, 15.79) |

### Example 2

**Table 3. Rates of Aggression Remission (R-MOAS≤ 10, LOCF), Low Weight Group (<30kg)**

| Remission Rate at End of Study | | Treatment group | | | |
|---|---|---|---|---|---|
| | | Placebo | Low Dose | Medium Dose | High Dose |
| Number of Patients | | 12 | 12 | 15 | 14 |
| End of Study (Visit 10), n(%) | | 3 (25.0) | 4 (33.3) | 4 (26.7) | 3 (21.4) |
| Treatment vs Placebo | Odds Ratio | | 1.53 | 1.36 | 0.62 |
| | (95% CI) | | (0.24, 9.60) | (0.22, 8.26) | (0.09,4.18) |
| | P-Value | | 0.648 | 0.738 | 0.623 |

**Table 4. Rates of Aggression Remission (R-MOAS≤ 10, LOCF), High Weight Group (≥30kg)**

| Remission Rate at End of Study | | Treatment group | | | |
|---|---|---|---|---|---|
| | | Placebo | Low Dose | Medium Dose | High Dose |
| Number of Patients | | 18 | 15 | 15 | 17 |
| End of Study (Visit 10), n(%) | | 3 (16.7) | 10 (66.7) | 8 (53.3) | 7 (41.2) |
| Treatment vs Placebo | Odds Ratio | | 12.08 | 7.07 | 4.07 |
| | (95% CI) | | (2.22, 65.62) | (1.35, 36.98) | (0.82, 20.27) |
| | P-Value | | 0.004 | 0.021 | 0.086 |

## Claims

1. A method of treating aggression in a human subject suffering from ADHD, comprising:
(a) determining the weight of the human subject;
(b) calculating a dose of molindone such to achieve a plasma concentration, based on body weight, that does not saturate the molindone receptors;
(c) administering the dose of step (b) to the mammalian subject.

2. The method according to claim 1, wherein the molindone receptors comprise D5 receptors.

3. The method according to claim 1, wherein the dose calculated occupies less than 90 % of the molindone receptors.

4. The method according to claim 1, wherein the dose calculated occupies less than 80 % of the molindone receptors.

5. The method according to claim 1, wherein the dose calculated occupies less than 70 % of the molindone receptors.

6. The method according to claim 1, wherein the dose calculated occupies less than 50 % of the molindone receptors.

7. A method of treating aggression in a human subject suffering from ADHD, comprising administering a daily dose of molindone between 15 mg and 60 mg for human subjects weighing over 30 kg and a daily dose of molindone less than 25mg for human subjects weighing less than 30 kg.
